# EUROPÄISCHE PATENTSCHRIFT

(11) **EP 3 097 938 B1**
(45) Veröffentlichungstag und Bekanntmachung des Hinweises auf die Patenterteilung: **08.07.2020**
(21) Anmeldenummer: 16170503.3
(22) Anmeldetag: 19.05.2016
(51) Int. Cl.: A61M 16/12, A61M 16/20

(54) **VORRICHTUNG ZUR APPLIKATION EINES MEDIZINISCHEN GASES AN EINEN PATIENTEN**
DEVICE FOR APPLYING A MEDICAL GAS TO A PATIENT
DISPOSITIF D'ADMINISTRATION D'UN GAZ MEDICAL A UN PATIENT

(30) Priorität: 26.05.2015 DE 102015108283
(43) Veröffentlichungstag der Anmeldung: 30.11.2016
(73) Patentinhaber: EKU Elektronik GmbH, 56291 Leiningen (DE)
(72) Erfinder: Ulrichskötter, Hermann-Josef, 79098 Freiburg (DE); Köbrich, Rainer, 68809 Neulussheim (DE); Erdmann, Hans, 56291 Leiningen (DE)
(74) Vertreter: von Bülow & Tamada

(56) Entgegenhaltungen:
- DE-A1- 19 961 206
- DE-A1-102010 016 699
- US-A- 5 615 669
- US-A1- 2005 076 907

## Beschreibung

Die vorliegende Erfindung betrifft eine Vorrichtung zur Applikation eines medizinischen Gases, auch Therapiegas genannt, an einen Patienten und ein Verfahren zur Ansteuerung eines Stellgliedes in der Vorrichtung.

Aus der EP 0 960 629 B1 ist eine Vorrichtung zur Applikation oder Verabreichung eines medizinischen Gases in Form von Stickstoffmonoxid an einen Patienten, bekannt, die einen als Rohrleitung ausgebildeten Atemgaskanal, der eingerichtet ist, einen Atemgasfluss zum Patienten zu führen, einen das medizinische Gas von einer Gasquelle in den Atemgasfluss führenden Applikationskanal, und ein das medizinische Gas im Applikationskanal in Abhängigkeit wenigstens eines Steuersignals dosierendes Dosierstellglied umfasst. Das Steuersignal wird von einer Logikschaltung erzeugt, wenn in der Rohrleitung der Druck negativ wird. Dann wird vom Beginn der Einatmung des Patienten ausgegangen. Auf diese Weise kann das medizinische Gas zu Beginn der Einatmung zeitlich präzise dosiert werden.

Die DE 10 2010 016 699 A1 offenbart eine Vorrichtung zur Applikation eines medizinischen Gases an einen Patienten, umfassendeinen Atemgaskanal, der eingerichtet ist, einen Atemgasfluss zum Patienten zu führen, einen das medizinische Gas von einer Gasquelle in den Atemgasfluss führenden Applikationskanal und ein das medizinische Gas im Applikationskanal in Abhängigkeitwenigstens eines Steuersignals dosierendes Dosierstellglied.

Die US 2005/0076907 A1 offenbart eine Vorrichtung gemäß dem Oberbegriff des geltenden Anspruchs 1.

Es ist Aufgabe der vorliegenden Erfindung, die bekannte Vorrichtung zu verbessern.

Die Aufgabe wird durch die Merkmale der unabhängigen Ansprüche gelöst. Bevorzugte Weiterbildungen sind Gegenstand der abhängigen Ansprüche.

Gemäß einem Aspekt der Erfindung umfasst eine Vorrichtung zur Applikation eines medizinischen Gases an einen Patienten einen Atemgaskanal, der eingerichtet ist, einen Atemgasfluss zum Patienten zu führen, einen das medizinische Gas von einer Gasquelle in den Atemgasfluss führenden Applikationskanal, ein das medizinische Gas im Applikationskanal in Abhängigkeit wenigstens eines Steuersignals dosierendes Dosierstellglied, eine Erfassungseinrichtung, die eingerichtet ist, ein Profil einer Kenngröße des Atemgasflusses im Atemgaskanal zu erfassen und eine Steuereinrichtung, die eingerichtet ist, das Steuersignal in Abhängigkeit des erfassten Profils zu erzeugen. Das Profil der Kenngröße ist bevorzugt der zeitliche Verlauf der Kenngröße.

Der angegebenen Vorrichtung liegt die Überlegung zugrunde, dass die Dosierung des medizinischen Gases nicht nur zeitlich sondern auch örtlich präzise in der Lunge des Patienten erfolgen sollte, damit das medizinische Gas exakt an der Stelle angreift, an der eine Behandlung mit dem medizinischen Gas erforderlich ist. Hierzu sind jedoch Kenntnisse zu den geometrischen Verhältnissen der Lunge des Patienten notwendig, damit das medizinische Gas ab einem bestimmten Füllgrad der Lunge mit eingeatmeter Luft in die Atmung des Patienten eingeleitet wird.

Hier nutzt die angegebene Vorrichtung die Erkenntnis, dass sich aus dem Atemgasfluss Rückschlüsse auf die vom Patienten eingeatmete Atemluft ziehen lassen. Aus dem Profil des Atemgasflusses kann in charakteristischer Weise der Füllgrad der Lunge mit eingeatmeter Luft abgeleitet werden. Dass heißt, dass basierend auf dem Profil des Atemgasflusses das medizinische Gas in Abhängigkeit des Füllgrades der Lunge in den Atemgasfluss eingebracht werden kann. Wird das medizinische Gas erst ab einem bestimmten Füllgrad in den Atemgasfluss eingebracht, dann wirkt es im Bereich dieses Füllgrades besonders konzentriert. Das heißt, dass durch Ansteuern des Dosierstellgliedes mit dem Steuersignal in Abhängigkeit des Profils des Atemgasflusses das medizinische Gas gezielt an bestimmte Stellen in der Lunge des Patienten gebracht werden kann, wodurch sich die medizinische Behandlung des Patienten durch Verwendung der angegebenen Vorrichtung verbessern lässt.

Grundsätzlich kann als Kenngröße des Atemgasflusses eine beliebige Kenngröße verwendet werden. So wäre es beispielsweise möglich, als Kenngröße den Druck im Atemgaskanal zu erfassen, weil dieser sich durch die Atmung des Patienten im Atemgaskanal in charakteristischer Weise ändert und so ein Profil aufweist, basierend auf dem auf den oben genannten Füllgrad der Lunge geschlossen werden kann. Vorzugsweise umfasst die Erfassungsvorrichtung der angegebenen Vorrichtung einen Strömungssensor zum Erfassen einer Strömung des Atemgasflusses des Atemgasflusses. Der Weiterbildung liegt die Überlegung zugrunde, dass ein Drucksensor den tatsächlichen Zustand der Inspirationsphase nur unzureichend genau ermitteln kann, weil der eigentliche, für die Inspirationsphase interessierende Atemgasfluss erst die Folge eines von Patienten durch seine Atmung aufgebauten Druckes ist. Entsprechende Zeitverschiebungen bei der Applikation des medizinischen Gases bei der Verwendung eines Drucksensors müssen dann aufwändig korrigiert werden. Derartige Korrekturen sind bei der direkten Erfassung des Atemgasflusses durch die vorgeschlagene Verwendung eines Strömungssensors nicht notwendig.

Zur weiteren Steigerung der Genaugigkeit bei der Erfassung des Zustandes der Inspirationsphase kann die Steuereinrichtung eingerichtet sein, das Steuersignal basierend auf einer Fusion des erfassten Profils mit wenigstens einer weiteren, eine Inspiration des Patienten beschreibenden Information auszugeben. Unter einer Fusion von Informationen soll nachstehend ein Verfahren verstanden werden, das Daten aus unterschiedlichen Sensoren oder Informationsquellen verknüpft, mit dem Ziel, neues und präziseres Wissen über die Inspiration des Patienten zu gewinnen. Hierbei stellt ein Teil der zu fusionierenden Informationen das erfasste Profil dar, während ein anderer Teil der zu fusionierenden Informationen aus einer anderen beliebigen Informationsquelle über die Inspiration des Patienten stammen kann. So kann diese andere Informationsquelle beispielsweise ein weiterer Sensor, ein Simulationsmodell oder dergleichen sein. Fusioniert werden können die angegebenen Informationen dann in einer beliebigen Fusionseinrichtung, wie beispielsweise einem Kalmann-Filter.

In einer anderen Weiterbildung der angegebenen Vorrichtung kann die Steuereinrichtung eingerichtet sein, basierend auf dem Profil eine Bedingung für die Kenngröße des Atemgasflusses zu bestimmen und das Steuersignal in Abhängigkeit einer Gegenüberstellung der bestimmten Bedingung und der Kenngröße des Atemgasflusses zu erzeugen. Dieser Weiterbildung liegt die Überlegung zugrunde, dass die volumetrische Aufnahmefähigkeit der Lunge eines Patienten an Atemgas vom Körperbau des Patienten abhängig ist, der sich jedoch von Patient zu Patient unterscheidet. Zwar wäre es möglich die volumetrische Aufnahmefähigkeit vor der Behandlung des Patienten beispielsweise durch Programmierung vorzugeben, durch das Profil des Atemgasflusses jedoch liegen alle notwendigen Informationen zur Bestimmung der volumetrischen Aufnahmefähigkeit der Lunge vor, so dass diese automatisch bestimmt werden kann, ohne dass der Patient vor der Behandlung beispielsweise durch Röntgen erst aufwändig vermessen werden muss.

In einer besonderen Weiterbildung der angegebenen Vorrichtung ist die Steuereinrichtung eingerichtet, bezogen auf die Atmung des Patienten in einer Periode des Atemgasflusses die Bedingung zu bestimmen und in einer darauf folgenden Periode des Atemgasflusses die bestimmte Bedingung zur Erzeugung des Steuersignals zu verwenden.

In einer anderen Weiterbildung umfasst die angegebene Vorrichtung nach einem der vorstehenden Ansprüche einen Expirationskanal, der eingerichtet ist, den Atemgasfluss vom Patienten wegzuführen und eine weitere Erfassungseinrichtung, die eingerichtet ist, einen Gehalt an medizinischem Gas im Atemgasfluss des Expirationskanals zu erfassen, wobei die Steuereinrichtung, eingerichtet ist, das Steuersignal in Abhängigkeit des erfassten Gehalts an medizinischem Gas im Atemgasfluss zu erzeugen. Der Weiterbildung liegt die Überlegung zugrunde, dass die Behandlung des Patienten zusätzlich durch eine Sicherstellung optimiert werden könnte, dass in der Lunge eine bestimmte Menge an medizinischem Gas vom Körper des Patienten aufgenommen und/oder verarbeitet wird. In diesem Fall wird dieses medizinische Gas selbstverständlich beim Ausatmen nicht wieder ausgeatmet. Daher ist der Gehalt am medizinischem Gas im ausgeatmeten Atemgas eine geeignete Messgröße für das vom Körper in der Lunge aufgenommene medizinische Gas. Mit der Steuereinrichtung kann durch ein geeignetes Einstellen des Steuersignals nun entweder versucht werden unnötige Übersättigungen bei der Applikation des medizinischen Gases zu vermeiden und/oder die Menge an zu verabreichendem medizinischen Gas auf einen bestimmten Sollwert einzustellen.

Hierzu kann die Steuereinrichtung der angegebenen Vorrichtung in einer zusätzlichen Weiterbildung eingerichtet sein, das Steuersignal derart zu erzeugen, dass die Menge des medizinischen Gases im Atemgasfluss durch den Expirationskanal an einen vorbestimmten Sollwert angeglichen wird. Dabei kann der Atemgaskanal zweckmäßigerweise ein den Atemgasfluss zum Patienten führender Inspirationskanal und die Steuereinrichtung eingerichtet sein, den Sollwert in Abhängigkeit einer Gegenüberstellung dem Gehalt des medizinischen Gases im Atemgasfluss durch den Expirationskanal und einer Menge des medizinischen Gases im Atemgasfluss durch den Inspirationskanal einzustellen. Die Menge des medizinischen Gases im Atemgasfluss durch den Inspirationskanal kann hierbei beispielsweise unmittelbar aus der Ansteuerung des Dosierstellgliedes abgeleitet werden. Mit der vorliegenden Weiterbildung wird sichergestellt, dass in der Lunge des Patienten genau die Menge an medizinischen Gas aufgenommen wird, die auch an den Patienten appliziert werden soll.

In einer besonderen Weiterbildung der angegebenen Vorrichtung die Steuereinrichtung eingerichtet ist, das Steuersignal als Puls auszugeben. Dabei kann das Stellglied wenigstens ein Schaltmittel umfassen, das eingerichtet ist, den Applikationskanal basierend auf dem Puls zu öffnen und schließen. Durch die pulsartige Erzeugung des Steuersignals in Abhängigkeit des Profils des Atemflusses des Patienten wird das Dosierstellglied gezielt aktiviert und deaktiviert. Auf diese Weise kann die oben erläuterte örtliche Dosierung des medizinischen Gases weiter verbessert werden, weil die Zugabe an medizinischen Gas ab einen bestimmten Füllgrad der Lunge mit Luft auch wieder abgestellt werden kann. Alternativ oder zusätzlich kann das Dosierstellglied der angegebenen Vorrichtung eine Bank aus Schaltmitteln umfassen, wobei die Steuereinrichtung eingerichtet ist, das Steuersignal mehrdimensional zur individuellen Ansteuerung der einzelnen Schaltmittel der Bank auszugeben. Hierbei kann mit jedem Schaltmittel ein bestimmtes Dosierverhalten beim Dosieren des medizinischen Gases vorgegeben werden, wobei im Betrieb dann für ein gewünschtes Dosierverhalten dann das entsprechende Schaltmittel angesteuert werden kann.

In einer anderen Weiterbildung der angegebenen Vorrichtung besitzen die einzelnen Schaltmittel des Stellgliedes voneinander verschiedene Dosierverhalten für das medizinische Gas.

In einer noch anderen Weiterbildung der angegebenen Vorrichtung umfasst das Stellglied eine in Reihe zum Schaltmittel geschaltete Drosselblende. Durch die Drosselblende kann der Atemgaskanal unabhängig von der Ansprechzeit der Schaltmittel verschlossen werden, was Totzeiten weiter reduziert.

Gemäß einem weiteren Aspekt der Offenbarung umfasst ein Verfahren zur Ansteuerung eines Stellgliedes in einer angegebenen Vorrichtungen die Schritte Erfassen eines Profil einer Kenngröße des Atemgasflusses im Atemgaskanal und Erzeugen des Steuersignals in Abhängigkeit des erfassten Profils.

Gemäß einem weiteren Aspekt der Erfindung ist eine Steuervorrichtung eingerichtet das angegebene Verfahren durchzuführen.

In einer Weiterbildung der angegebenen Vorrichtung weist die angegebene Vorrichtung einen Speicher und einen Prozessor auf. Dabei ist das angegebene Verfahren in Form eines Computerprogramms in dem Speicher hinterlegt und der Prozessor zur Ausführung des Verfahrens vorgesehen, wenn das Computerprogramm aus dem Speicher in den Prozessor geladen ist.

Gemäß einem weiteren Aspekt der Erfindung umfasst ein Computerprogramm Programmcodemittel, um alle Schritte des angegebenen Verfahrens durchzuführen, wenn das Computerprogramm auf einem Computer oder einer der angegebenen Vorrichtungen ausgeführt wird.

Gemäß einem weiteren Aspekt der Erfindung enthält ein Computerprogrammprodukt einen Programmcode, der auf einem computerlesbaren Datenträger gespeichert ist und der, wenn er auf einer Datenverarbeitungseinrichtung ausgeführt wird, des angegebenen Verfahrens durchführt.

Gemäß einem weiteren Aspekt der Erfindung umfasst eine Vorrichtung zur Applikation eines medizinischen Gases an einen Patienten einen Inspirationskanal, der eingerichtet ist, einen Inspirationsgasfluss zum Patienten zu führen, einen Expirationskanal, der eingerichtet ist, einen Inspirationsgasfluss vom Patienten weg zu führen, einen das medizinische Gas von einer Gasquelle in den Atemgasfluss führenden Applikationskanal, ein das medizinische Gas im Applikationskanal in Abhängigkeit wenigstens eines Steuersignals dosierendes Dosierstellglied, eine Erfassungseinrichtung, die eingerichtet ist, einen Gehalt an medizinischem Gas im Expirationskanal zu erfassen, und eine Steuereinrichtung, die eingerichtet ist, das Steuersignal in Abhängigkeit dem erfassten Gehalt zu erzeugen.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit der Zeichnung näher erläutert werden. Es zeigen:
- Fig. 1: eine Prinzipdarstellung einer Vorrichtung zur Applikation eines medizinischen Gases an einen Patienten,
- Fig. 2: eine schematische Darstellung der Vorrichtung aus Fig. 1,
- Fig. 3a: ein Diagramm mit einem Messsignal in der Vorrichtung der Fig. 1,
- Fig. 3b: ein Diagramm mit einer von der Vorrichtung der Fig. 1 abgegebenen Menge an medizinischem Gas über die Zeit,
- Fig. 4: eine weitere schematische Darstellung Beatmungsgerätes in der Vorrichtung aus Fig. 1, und
- Fig. 5: eine schematische Darstellung einer Weiterbildung der Vorrichtung aus Fig. 2.

In den Figuren werden gleiche technische Elemente mit gleichen Bezugszeichen versehen und nur einmal beschrieben. Die Figuren sind rein schematisch und geben vor allem nicht die tatsächlichen geometrischen Verhältnisse wieder.

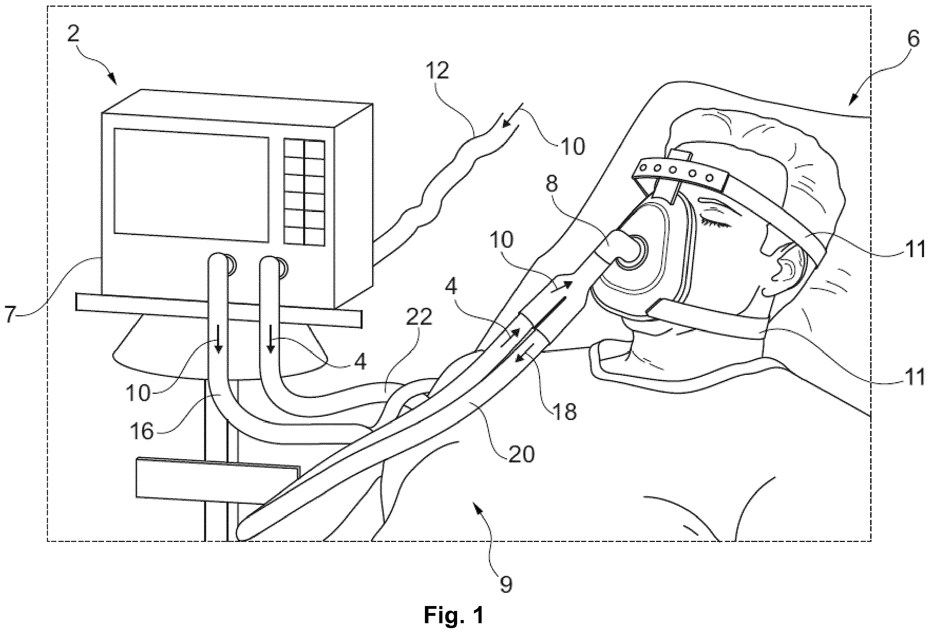

Bezugnehmend auf Fig. 1 wird nachstehend eine Vorrichtung 2 zur Applikation eines medizinischen Gases 4 an einen Patienten 6 beschrieben. Die Vorrichtung 2 ist als Gerät zur Atemgasdosierung bei der Spontanatmung ausgeführt, kann aber auch ein bei der Atmung assistierendes Beatmungsgerät, wie beispielsweise bei der CPAP-Beatmung (Continuous Positive Airway Pressure Beatmung genannt) verwendet, sein.

Die Vorrichtung 2 besteht im Wesentlichen aus einem Beatmungsgerät 7 zur Ausgabe von Atemluft und des medizinischen Gases 4 sowie einem Schlauchsystem 9 zum Transport der Atemluft und des medizinischen Gases 4 zum Patienten 6. Als Atemluft kann beispielsweise ein Gemisch aus Sauerstoff und Stickstoff verwendet werden, während das medizinische Gas in Abhängigkeit einer Therapie des Patienten 6 wählbar ist. Ein Beispiel für ein mögliches medizinische Gas 4 wäre Stickstoffmonoxid oder eine Mischung aus Stickstoffmonoxid und Stickstoff, worauf aber nicht weiter eingegangen werden soll.

Das Schlauchsystem 9 weist ein totraumreduzierendes Interface 8 in Form einer Atemmaske auf, über das an den Patienten 6 die einzuatmende Atemluft als nachstehend Inspirationsgasfluss 10 genannter Atemgasfluss angelegt wird. Alternativ kann das totraumreduzierende Interface 8 auch ein Mundstück, eine Nasenbrille oder ein Tubus sein. Das totraumreduzierende Interface 8 ist in der vorliegenden Ausführung mit Riemen 11 am Kopf des Patienten befestigt.

Der Inspirationsgasfluss 10 wird aus einer nicht weiter dargestellten Luftquelle bereitgestellt und passiert über einen Versorgungsschlauch 12 das noch zu beschreibende Beatmungsgerät 7. Dieses gibt den Inspirationsgasfluss 10 an einen Atemgaskanal in Form eines schlauchförmigen Inspirationskanals 16 ab, der den Inspirationsgasfluss 10 in das totraumreduzierende Interface 8 führt.

Der Patient 6 atmet dann die Atemluft aus dem Inspirationsfluss 10 über das totraumreduzierende Interface 8 ein und atmet anschließend wieder Atemluft als einen weiteren, Expirationsgasfluss 18 genannten Atemgasfluss aus. Der Expirationsgasfluss 18 strömt abschließend über einen weiteren Atemgaskanal in Form eines schlauchförmigen Expirationskanals 20 in die Umgebung.

Das medizinische Gases 4 wird zur Applikation an den Patienten 6 vom Beatmungsgerät 7 über einen schlauchförmigen Applikationskanal 22, auch Patientenzuleitung genannt, ausgegeben und in den Inspirationskanal 16 geleitet. Hierbei kann am Applikationskanal 22 ein Absperrhahn 23 angeordnet sein, um die Applikation des medizinischen Gases 6 mechanisch zu unterbrechen.

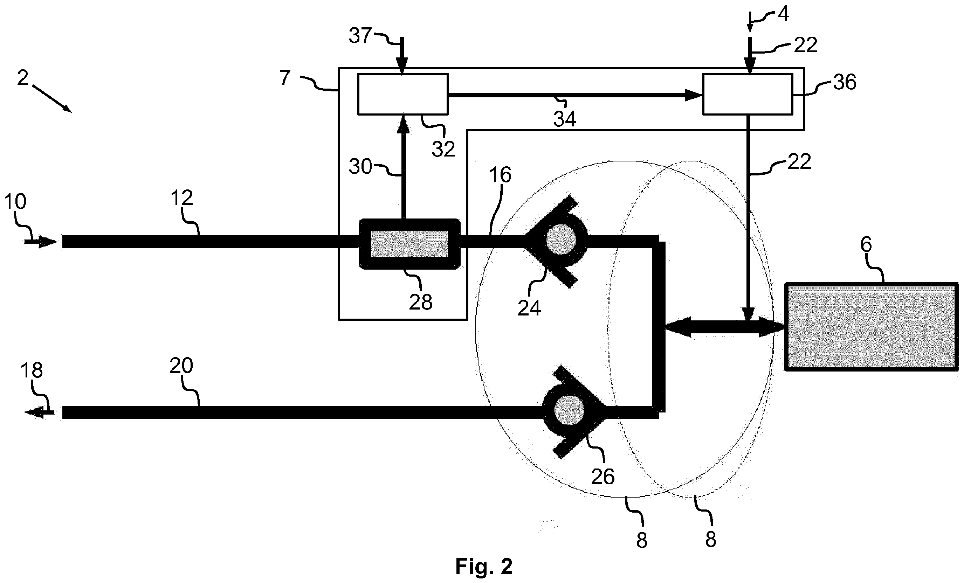

Es wird auf Fig. 2 Bezug genommen, die die Vorrichtung 2 aus Fig. 1 in einer schematischen Darstellung zeigt.

Damit der Patient 6 nicht durch den Expirationskanal 20 inhaliert und durch den Inspirationskanal 16 exhaliert, sind entsprechend im Inspirationskanal 16 ein Inspirationsrückschlagventil 24 und im Expirationskanal ein Expirationsrückschlagventil 26 angeordnet. Das Inspirationsrückschlagventil 24 ist im Inspirationskanal 16 derart angeordnet, dass der Inspirationsgasfluss 10 zum Patientien 6 hin ungehindert strömen kann, ein entgegengesetzter Gasstrom jedoch blockiert wird. Demgegenüber ist das Expirationsrückschlagventil 26 im Expirationskanal 20 derart angeordnet, dass der Expirationsgasfluss 18 ungehindert vom Patienten 6 wegströmen kann, ein Gasstrom zum Patienten 6 hin jedoch blockiert wird.

Atmet der Patient 6 ein, so entsteht im totraumreduzierenden Interface 8 ein Unterdruck. Dieser Unterdruck öffnet das Inspirationsrückschlagventil 24 und verschließt das Expirationsrückschlagventil 26, so dass der Inspirationsgasfluss 10 über den Inspirationskanal 16 zum Patienten 6 hinströmen kann. Atmet der Patient 6 demgegenüber aus, so entsteht im totraumreduzierenden Interface 8 ein Überdruck, der das Expirationsrückschlagventil 26 öffnet und das Inspirationsrückschlagventil 24 verschließt. Auf diese Weise kann der Expirationsgasfluss 18 ungehindert über den Expirationskanal 20 vom Patienten 6 wegströmen und entweichen kann.

Wie in Fig. 2 angedeutet, können die beiden Rückschlagventile 24, 26 optional im totraumreduzierenden Interface 8 integriert sein. Wie ebenfalls in Fig. 2 angedeutet, kann auch das medizinische Gas 4 statt in den Inspirationskanal 22 direkt in das totraumreduzierende Interface 8 eingeführt werden.

Nachstehend wird die Dosierung des medizinischen Gases 4 durch das Beatmungsgerät 7 erläutert.

Zur Dosierung des medizinischen Gases 4 erfasst das Beatmungsgerät 7 mit einer Erfassungseinrichtung 28 eine physikalische Größe des Atemgasflusses 16, 20 des Patienten 6. Zwar kann die physikalische Größe eines beliebigen Atemgasflusses erfasst werden, in der vorliegenden Ausführung erfasst das Beatmungsgerät eine physikalische Größe des Inspirationsgasflusses 10. Als physikalische Größe kann jede beliebige, den Atemgasfluss 16, 20 des Patienten 6 zeitlich beschreibende physikalische Größe gewählt werden, wie beispielsweise ein Druck des Atemgasflusses 16, 20. In der vorliegenden Ausführung erfasst das Beatmungsgerät 7 als physikalische Größe eine Strömung in einem Strömungsmesssignal 30 des Inspirationsgasflusses 10, wofür die Erfassungseinrichtung 28 einen nicht weiter gezeigten Strömungssensor umfasst oder als dieser ausgebildet ist. Zur Strömungsmessung kann beispielsweise eine Differenzdruck-Messung oder eine Hitzedrahtanamomentrie zum Einsatz kommen.

Das Beatmungsgerät 7 umfasst ferner eine Steuereinrichtung 32, die das Strömungsmesssignal 30 mit der erfassten Strömung empfängt, basierend darauf in einer noch zu beschreibenden Weise ein Steuersignal 34 erzeugt und mit dem erzeugten Steuersignal 34 ein Dosierstellglied 36 ansteuert, mit dem das medizinische Gas 4 im Applikationskanal 22 dosiert wird. Das dosierte medizininische Gas 4 wird dann in der bereits erläuterten Weise in den Inspirationsgasfluss 10 geleitet, um es an den Patienten 6 zu applizieren.

Ziel der Applikation des medizinische Gas 4 an den Patienten ist es, dass das medizinische Gas 4 an bestimmten Zielstellen seiner Lunge einen therapeutischen Effekt auslöst. Weil die Zielstellen in der Lunge des Patienten 6 in der Regel nicht direkt dem medizinischen Gas 4 ausgesetzt werden können, könnte die Lunge des Patienten 6 möglichst vollständig mit dem medizinischen Gas 4 ausgefüllt werden. Dann wäre sichergestellt, dass alle Stellen in der Lunge (also auch die Zielstelle) dem medizinischen Gas 4 ausgesetzt sind.

Die zuvor erläuterte Applikation hat jedoch zwei entscheidende Nachteile. Die Menge an hierzu in die Lunge des Patienten einzuführenden medizinischen Gases 4 ist vergleichsweise hoch, was zu einer Verschwendung an medizinischem Gas 4 führt. Weit nachteiliger ist jedoch, dass die Konzentration an medizinischen Gas 4 in der Lunge begrenzt ist. Der Patient kann in der Regel nicht einer beliebig hohen Konzentration an medizinischem Gas 4 ausgesetzt werden, beispielsweise wenn als medizinisches Gas 4 das oben genannte Stickstoffmonoxid gewählt wird.

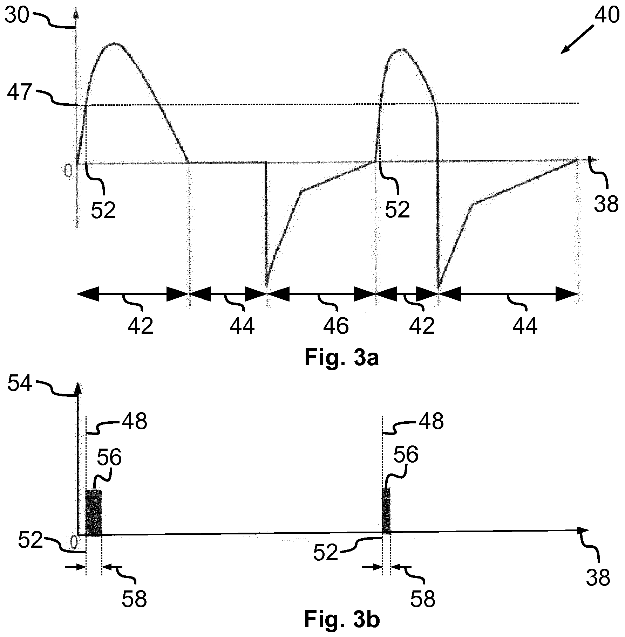

Um diese Nachteile zu vermeiden wird in der Vorrichtung 2 der vorliegenden Ausführung vorgeschlagen, die Dosiereinrichtung 36 derart mit der Steuereinrichtung 32 anzusteuern, dass das medizinische Gas 4 an Zielstellen gemäß einer Therapievorgabe 37 in der Lunge transportiert wird. Dieser Vorschlag soll nachstehend anhand von Fig. 3a erläutert werden, die ein Diagramm mit dem Strömungsmesssignal 30 über die Zeit 38 zeigt.

Das Strömungsmesssignal 30 weist ein die Atmung des Patienten 6 kennzeichnendes Profil 40 auf, das grundsätzlich in drei Phasen unterteilt werden kann. Während einer Inspirationsphase 42 strömt Atemluft zum Patienten 6 und das Strömungsmesssignal 30 ist positiv. An die Inspirationsphase 42 schließt sich eine Atemstillstandsphase 44 an, in der der Patient 6 keine Atemluft bewegt und das Strömungsmesssignal 30 Null ist. Daran schließt sich eine Expirationsphase 46 an, in Atemluft vom Patienten wegströmt und das Strömungsmesssignal 30 negativ ist. An die Expirationsphase 46 schließt sich eine neue Inspirationsphase 42 an und die Atmung beginnt von vorn.

Um nun das medizinische Gas 4 gezielt an die Zielstellen gemäß der Therapievorgabe 37 in der Lunge zu transportieren, stellt die Steuereinrichtung 32 zur Erzeugung des Steuersignals 34 aus dem Strömungsmesssignal 30 das Strömungsmesssignal 30 einer vorbestimmten Bedingung 47 gegenüber und gibt als Steuersignal 34 in Fig. 3b durch gepunktete Linien angedeutete Pulse 48 aus, wenn das Strömungsmesssignal 30 die vorbestimmte Bedingung erfüllt.

Die vorbestimmte Bedingung 47 stellt ein Kriterium dar, ab wann die Lunge während er Inspirationsphase 42 bis zur Zielstelle gemäß der Therapievorgabe 37 mit Atemluft gefüllt ist. Ab diesem Moment wird das medizinische Gas 4 eingeleitet und greift damit unmittelbar an der Zielstelle gemäß der Therapievorgabe 37 in der Lunge an.

Die Therapievorgabe 37 kann beispielsweise von einem behandelnden Arzt der Steuereinrichtung 32 am Behandlungsgerät 7 vorgegeben werden. Als geeignete Therapievorgabe 37 wäre beispielsweise denkbar, dass der Arzt vorgibt in welcher prozentualen Höhe sich die Zielstelle ausgehend von der Gesamthöhe der Lunge befindet, wovon nachstehend als Beispiel ausgegangen werden soll.

Zur Bestimmung der vorbestimmten Bedingung 47 kann die Steuereinrichtung 32 hierzu das Profil des Strömungsmesssignals 30 verwenden. Hierzu bestimmt die Steuereinrichtung 32 zunächst während einer der der Inspirationsphasen 42 durch Integration des Strömungsmesssignals 30 das Gesamtvolumen der Lunge des Patienten. Anschließend kann die Steuereinrichtung 32 dann basierend auf dem gemessenen Gesamtvolumen und der mit der Theraphievorgabe 37 gemachten Höhen ein Füllvolumen der Lunge bestimmen, ab dem das medizinische Gas 4 in den Atemgasfluss eingeleitet werden muss, um an der Zielstelle gemäß der Therapievorgabe 37 anzugreifen. Diesem Füllvolumen kann die Steuereinrichtung 32 abschließend als vorbestimmte Bedingung 47 einen ganz bestimmten Signalwert des Strömungsmesssignals 30 zuordnen.

Die Steuereinrichtung 32 kann nun das Steuersignal 34 derart generieren, dass die Pulse 48 in dem Steuersignal 34 zu Steuerzeitpunkten 52 ausgeben werden, an denen das Strömungsmesssignal 30 die vorbestimmte Bedingung 47 und damit den bestimmten Signalwert überschreitet.

Während des Betriebs des Beatmungsgerätes 7 kann die Steuervorrichtung 32 die vorbestimmte Bedingung 47 adaptiv verändern, um beispielsweise ein sich veränderndes Atemverhalten des Patienten zu berücksichtigen. Hierbei können auch Filter, wie ein Kalman-Filter zum Einsatz kommen, um Sprünge bei der Anpassung der vorbestimmten Bedingung zu vermeiden. Die vorbestimmte Bedingung kann dabei auch basierend auf einer Fusion von mehreren Sensorsignalen und nicht nur basierend auf dem einzelnen Strömungsmesssignal 30 bestimmt werden.

Nachstehend soll die Dosierung der medizinischen Gases 4 erläutert werden. Hierzu wird auf Fig. 3b Bezug genommen, die eine Dosiermenge 54 an medizinischem Gas 4 über die Zeit 38 zeigt.

Die von der Steuereinrichtung 32 in dem Steuersignal 34 ausgegebenen Pulse 48 lösen das Dosierstellglied 36 aus, das den Applikationskanal 22 öffnet, so dass das medizinische Gas in den Inspirationsstrom 10 geleitet wird.

Das Dosierstellglied 36 kann die Dosiermenge 54 an medizinischem Gas 4 in einer beliebigen Weise einstellen. In der vorliegenden Ausführung soll das Dosierstellglied 36 die Dosiermenge 54 an medizinischem Gas 4 derart einstellen, dass das medizinische Gas in Form von Gaspulsen 56 mit einer variablen Breite 58 in den Inspirationsgasfluss 10 eingeführt wird. Die Breite 58 kann beispielsweise basierend auf einer vom Patienten 6 aufgenommenen Menge an medizinischem Gas 4 eingestellt werden, worauf an späterer Stelle näher eingegangen wird.

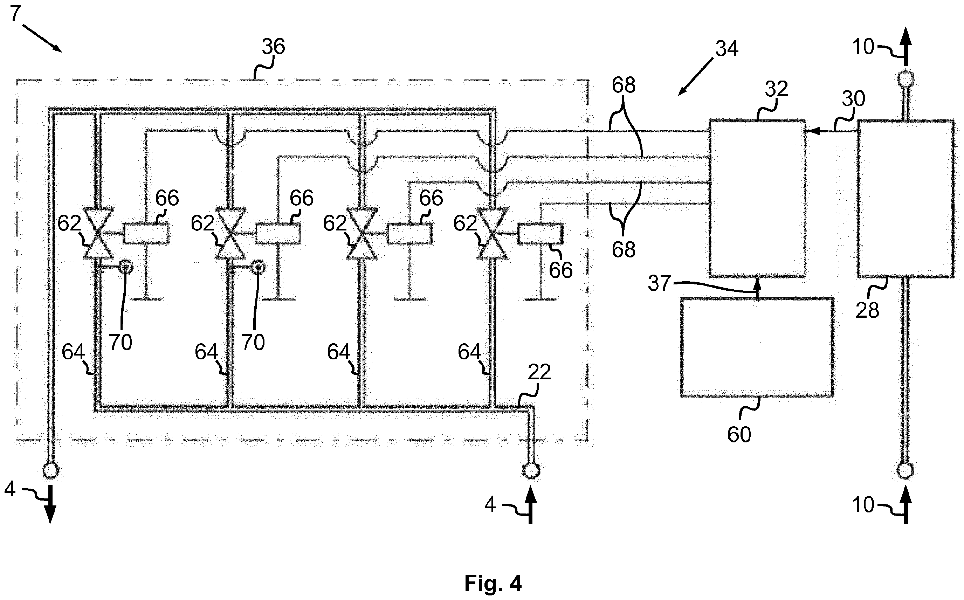

Zunächst soll anhand von Fig. 4 auf eine Ausführung des Dosierstellgliedes 36 näher eingegangen werden, mit der sich in einfacher Weise die variable Breite 58 für die Gaspulse 56 erreichen lässt. Fig. 4 zeigt eine schematische Darstellung des Beatmungsgerätes 7, wobei die Therapievorgabe 37 aus einem Speicher 60 bereitgestellt wird, der beispielsweise vom behandelnden Arzt zuvor mit der Therapievorgabe 37 programmiert werden kann.

In Fig. 4 weist das Dosierglied 36 des Beatmungsgerätes 7 mehrere Schaltmittel in Form von Ventilen 62 auf. Ferner spaltet sich der Applikationskanal 22 in mehrere Unterapplikationskanäle 64 auf, wobei der Durchlass in jedem Unterapplikationskanal 64 durch ein Schalten der entsprechenden Ventile 62 geöffnet beziehungsweise geschlossen werden kann. Die einzelnen Ventile 62 können sich in Ihren Schalteigenschaften unterscheiden. Auf Fig. 3b bezogen wären das die Dosiermenge 54 und der Breite 58 des Gaspulses 56.

Als besonders vorteilhaft hat es sich erwiesen, wenn eines der Ventile 62 mit einer Dosiermenge 54 von 0,1 l/min und ein weiteres Ventil 62 mit einer Dosiermenge 54 von 5 l/min dosiert. Ferner sollten die Ventile 62 derart schalten, dass die Gaspulse 56 mit einer Breite 58 von weniger als 7 ms, vorzugsweise zwischen 4 ms bis 7 ms erzeugt werden.

Um die zuvor genannten hohen Schaltzeiten zu garantieren, können als Ventile 62 beispielsweise Magnetventile verwendet werden, wie durch Relais 66 geschaltet werden. Zur individuellen Ansteuerung der einzelnen Relais 66 kann das Steuersignal 34 als mehrdimensionales Steuersignal mit verschiedenen Signalkomponenten 68 ausgeführt sein. Die einzelnen Signalkomponenten 68 des Steuersignals 34 können grundsätzlich in verschiedenen Leitungen an die einzelnen Relais 66 übertragen werden. Alternativ wäre es auch möglich, an jedes einzelne Relais ein gemeinsames Steuersignal 34 anzulegen, in dem die einzelnen Signalkomponenten 68 überlagert sind.

Durch die parallele Anordnung der einzelnen Ventile 62 zu einer gemeinsamen Ventilbank lassen sich eine Vielzahl von verschiedenen Dosiermengen 54 einstellen. So kann grundsätzlich mit jedem Ventil 62 einzeln eine bestimmte Dosiermenge 54 aber auch in der Kombination mehrere Ventile 62 gemeinsam eine Dosiermenge 54 eingestellt werden. Werden beispielsweise die zuvor genannten beiden speziellen Ventile 62 gemeinsam geschalten, so lässt sich eine Dosiermenge 54 von 5,1 l/min einstellen.

Sollten die Schalteigenschaften insbesondere bei Ventilen 62 mit hohen Dosiermengen 54 nicht ausreichend sein, um die Gaspulse 56 mit den oben genannten Breiten 58 zu realisieren, können in Reihe zu den Ventilen 62 auch Drosselklappen 70 geschaltet werden, die den entsprechenden Unterapplikationskanal 64 mit einer hohen Schaltzeit wieder verschließen.

Die Breite 58 der Gaspulse 56 kann mit dem Dosierglied 36 grundsätzlich nach beliebigen Kriterien eingestellt werden. Beispielsweise kann die Breite 58 in der Therapievorgabe 37 vorgegeben werden.

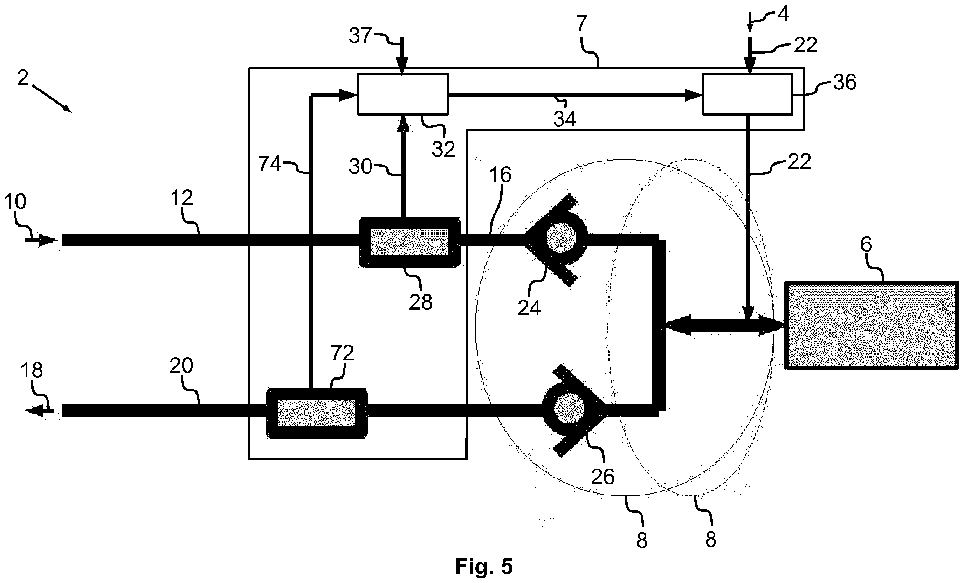

Eine alternative oder zusätzliche Möglichkeit, die Breite 58 der Gaspulse 56 einzustellen wird nachstehend anhand von Fig. 5 erläutert, die eine Weiterbildung der Vorrichtung 2 aus Fig. 2 zeigt.

In der weitergebildeten Vorrichtung 2 ist im Expirationskanal 20 eine weitere Erfassungsvorrichtung 72 angeordnet enthält. Die weitere Erfassungsvorrichtung 72 ermittelt im Expirationsfluss 18 einen Gehalt 74 an medizinischem Gas 4 und/oder an einem zu erwartenden Reaktionsprodukt, das der Patient basierend auf dem medizinischem Gas ausatmen müsste. Hierzu kann die weitere Erfassungsvorrichtung 72 dem Expirationskanal 20 einen Teil des ausgeatmeten Gases des Patienten 6 aus dem Expirationsfluss 18 entnehmen und in dem entnommenen Teil den oben genannten Gehalt 74 ermitteln.

Die Steuereinrichtung 32 kann diesen Gehalt 74 dann empfangen und durch Einstellen des Stellsignals 34 an einen Sollgehalt anpassen, der beispielsweise in der Therapievorgabe 37 mit vorgegeben werden kann. So kann sichergestellt werden, dass am Patient 6 auch tatsächlich eine vorbestimmte Menge an medizinischem Gas 4 wirkt.

Die Regelung des Gehaltes 74 an medizinischem Gas 4 und/oder an dem zu erwartenden Reaktionsprodukt kann auch unabhängig von der oben erläuterten Applikation des medizinischen Gases in einem bestimmten Bereich der Lunge des Patienten 6 realisiert werden. Hierbei ist der Strömungssensor 28 in Fig. 5 dann entsprechend obsolet.

## Patentansprüche

1. Vorrichtung (2) zur Applikation eines medizinischen Gases (4) an einen Patienten (6), umfassend:
- einen Atemgaskanal (12, 20), der eingerichtet ist, einen Atemgasfluss (10, 18) zum Patienten (6) zu führen,
- einen das medizinische Gas (4) von einer Gasquelle in den Atemgasfluss (10, 18) führenden Applikationskanal (22),
- ein das medizinische Gas (4) im Applikationskanal (22) in Abhängigkeit wenigstens eines Steuersignals (34) dosierendes Dosierstellglied (36), und
- eine Erfassungseinrichtung (28), die eingerichtet ist, ein Profil (40) einer Kenngröße (30) des Atemgasflusses (10, 18) im Atemgaskanal (12, 20) zu erfassen,
**gekennzeichnet durch**
- eine Steuereinrichtung (32), die eingerichtet ist, basierend auf dem erfassten Profil (40) einen Füllgrad einer Lunge des Patienten zu bestimmen und das Steuersignal (34) in Abhängigkeit des Füllgrades zu erzeugen.

2. Vorrichtung (2) nach Anspruch 1, wobei die Erfassungseinrichtung (28) einen Strömungssensor zum Erfassen einer Strömung des Atemgasflusses (10, 20) als Kenngröße umfasst.

3. Vorrichtung (2) nach Anspruch 2, wobei die Steuereinrichtung (32) eingerichtet ist, das Steuersignal () basierend auf einer Fusion des erfassten Profils (40) mit wenigstens einer weiteren, eine Inspiration des Patienten (6) beschreibenden Information auszugeben.

4. Vorrichtung (2) nach einem der vorstehenden Ansprüche, wobei die Steuereinrichtung (32) eingerichtet ist, basierend auf dem Profil (40) eine Bedingung (47) für die Kenngröße (30) des Atemgasflusses (10, 18) zu bestimmen und das Steuersignal (34) in Abhängigkeit einer Gegenüberstellung der bestimmten Bedingung (47) und der Kenngröße (30) des Atemgasflusses (10, 18) zu erzeugen.

5. Vorrichtung (2) nach Anspruch 4, wobei die Steuereinrichtung (32) eingerichtet ist, bezogen auf die Atmung des Patienten (6) in einer Periode (42) des Atemgasflusses (10, 18) die Bedingung (47) zu bestimmen und in einer darauf folgenden Periode (42) des Atemgasflusses (10, 18) die bestimmte Bedingung (47) zur Erzeugung des Steuersignals (34) zu verwenden.

6. Vorrichtung (2) nach einem der vorstehenden Ansprüche, umfassend:
- einen Expirationskanal (20), der eingerichtet ist, den Atemgasfluss (10, 18) vom Patienten wegzuführen, und
- eine weitere Erfassungseinrichtung (72), die eingerichtet ist, einen Gehalt (74) an medizinischem Gas (4) oder einem Reaktionsprodukt aus dem medizinischem Gas (4) im Atemgasfluss (18) durch den Expirationskanal (20) zu erfassen,
- wobei die Steuereinrichtung (32), eingerichtet ist, das Steuersignal (34) in Abhängigkeit des erfassten Gehalts (74) an medizinischem Gas (4) im Atemgasfluss (18) durch den Expirationskanal (20) zu erzeugen.

7. Vorrichtung (2) nach Anspruch 6, wobei die Steuereinrichtung (32) eingerichtet ist, das Steuersignal (34) derart zu erzeugen, dass die Menge des medizinischen Gases (4) im Atemgasfluss (18) durch den Expirationskanal (20) an einen vorbestimmten Sollwert angeglichen wird.

8. Vorrichtung (2) nach Anspruch 7, wobei der Atemgaskanal (12, 20) ein den Atemgasfluss (10, 18) zum Patienten (6) führender Inspirationskanal (12) und die Steuereinrichtung (32) eingerichtet ist, den Sollwert in Abhängigkeit einer Gegenüberstellung des Gehaltes (74) des medizinischen Gases (4) im Atemgasfluss (18) durch den Expirationskanal (20) und einer Menge des medizinischen Gases (4) im Atemgasfluss (10) durch den Inspirationskanal (12) einzustellen.

9. Vorrichtung (2) nach einem der vorstehenden Ansprüche, wobei die Steuereinrichtung (32) eingerichtet ist, das Steuersignal (34) als Puls (48) auszugeben und das Dosierstellglied (36) wenigstens ein Schaltmittel (62, 66) umfasst, das eingerichtet ist, den Applikationskanal (22) basierend auf dem Puls (48) für eine vorbestimmte Schaltdauer (58) zu öffnen.

10. Vorrichtung (2) nach Anspruch 9, wobei das Dosierstellglied (36) eine Bank aus Schaltmitteln (62, 66) umfasst, und die Steuereinrichtung (32) eingerichtet ist, das Steuersignal (34) mehrdimensional zur individuellen Ansteuerung der einzelnen Schaltmittel (62, 66) der Bank auszugeben.

11. Vorrichtung (2) nach Anspruch 10, wobei die einzelnen Schaltmittel (62, 66) des Dosierstellgliedes (36) voneinander verschiedene Dosierverhalten (56) für das medizinische Gas (4) aufweisen.

12. Vorrichtung (2) nach einem der vorstehenden Ansprüche 9 bis 11, wobei das Dosierstellglied (36) eine in Reihe zum Schaltmittel (62, 66) geschaltete Drosselblende (70) umfasst.

13. Computerprogramm enthaltend Programmcodemittel, um alle Schritte eines Verfahrens zur Ansteuerung eines Dosierstellgliedes (36) in einer Vorrichtung (2) nach einem der vorstehenden Ansprüche durchzuführen, wenn das Computerprogramm auf einem Computer oder einer der angegebenen Vorrichtungen ausgeführt wird, umfassend die Schritte:
- Erfassen eines Profil (40) einer Kenngröße (30) des Atemgasflusses (10, 18) im Atemgaskanal (12, 20),
- Bestimmen eines Füllgrades einer Lunge des Patienten basierend auf dem erfassten Profil (40),
- Erzeugen des Steuersignals (34) in Abhängigkeit des bestimmten Füllgrades.

14. Steuervorrichtung (2), die eingerichtet ist, ein Verfahren zur Ansteuerung eines Dosierstellgliedes (36) in einer Vorrichtung (2) nach einem der Ansprüche 1 bis 12 durchzuführen, umfassend die Schritte:
- Erfassen eines Profil (40) einer Kenngröße (30) des Atemgasflusses (10, 18) im Atemgaskanal (12, 20),
- Bestimmen eines Füllgrades einer Lunge des Patienten basierend auf dem erfassten Profil (40),
- Erzeugen des Steuersignals (34) in Abhängigkeit des bestimmten Füllgrades.

15. Computerprogrammprodukt enthaltend einen Programmcode, der auf einem computerlesbaren Datenträger gespeichert ist und der, wenn er auf einer Datenverarbeitungseinrichtung ausgeführt wird, ein Verfahren zur Ansteuerung eines Dosierstellgliedes (36) in einer Vorrichtung (2) nach einem der Ansprüche 1 bis 12 durchführt, umfassend die Schritte:
- Erfassen eines Profil (40) einer Kenngröße (30) des Atemgasflusses (10, 18) im Atemgaskanal (12, 20),
- Bestimmen eines Füllgrades einer Lunge des Patienten basierend auf dem erfassten Profil (40),
- Erzeugen des Steuersignals (34) in Abhängigkeit des bestimmten Füllgrades.

## Claims

1. Device (2) for applying a medical gas (4) to a patient (6), comprising:
- a breathing gas channel (12, 20) adapted to conduct a flow of breathing gas (10, 18) to the patient (6),
- an application channel (22) leading the medical gas (4) from a gas source into the respiratory gas flow (10, 18),
- a dosing actuator (36) dosing the medical gas (4) in the application channel (22) as a function of at least one control signal (34), and
- a detection device (28) which is arranged to detect a profile (40) of a characteristic quantity (30) of the respiratory gas flow (10, 18) in the respiratory gas channel (12, 20),
**characterised by**
- a control device (32) arranged to determine a degree of filling of a patient's lung based on the detected profile (40) and to generate the control signal (34) in dependence on the degree of filling.

2. Device (2) according to claim 1, wherein the detection device (28) comprises a flow sensor for detecting a flow of the respiratory gas flow (10, 20) as a characteristic quantity.

3. Device (2) according to claim 2, wherein the control device (32) is arranged to output the control signal () based on a fusion of the detected profile (40) with at least one further piece of information describing an inspiration of the patient (6).

4. Device (2) according to one of the above claims, wherein the control device (32) is arranged to determine a condition (47) for the characteristic quantity (30) of the respiratory gas flow (10, 18) based on the profile (40) and to generate the control signal (34) in dependence on a comparison of the determined condition (47) and the characteristic quantity (30) of the respiratory gas flow (10, 18).

5. Device (2) according to claim 4, wherein the control device (32) is arranged to determine the condition (47) in relation to the respiration of the patient (6) in a period (42) of the respiratory gas flow (10, 18) and to use the determined condition (47) in a subsequent period (42) of the respiratory gas flow (10, 18) to generate the control signal (34).

6. Device (2) according to one of the above claims, comprising:
- an expiration channel (20) arranged to lead the respiratory gas flow (10, 18) away from the patient, and
- a further detection device (72) which is arranged to detect a content (74) of medical gas (4) or a reaction product from the medical gas (4) in the respiratory gas flow (18) through the expiration channel (20),
- wherein the control means (32) is arranged to generate the control signal (34) in dependence on the detected content (74) of medical gas (4) in the respiratory gas flow (18) through the expiration channel (20).

7. Device (2) according to claim 6, wherein the control device (32) is arranged to generate the control signal (34) in such a way that the quantity of medical gas (4) in the respiratory gas flow (18) through the expiration channel (20) is adjusted to a predetermined desired value.

8. Device (2) according to claim 7, wherein the respiratory gas channel (12, 20) is an inspiration channel (12) leading the respiratory gas flow (10, 18) to the patient (6) and the control device (32) is arranged to adjust the desired value in dependence on a comparison of the content (74) of the medical gas (4) in the respiratory gas flow (18) through the expiration channel (20) and a quantity of the medical gas (4) in the respiratory gas flow (10) through the inspiration channel (12).

9. Device (2) according to one of the above claims, wherein the control device (32) is arranged to output the control signal (34) as a pulse (48) and the dosing actuator (36) comprises at least one switching means (62, 66) arranged to open the application channel (22) based on the pulse (48) for a predetermined switching period (58).

10. Device (2) according to claim 9, wherein the metering actuator (36) comprises a bank of switching means (62, 66), and the control device (32) is arranged to output the control signal (34) multidimensionally for individual activation of the individual switching means (62, 66) of the bank.

11. Device (2) according to claim 10, wherein the individual switching means (62, 66) of the metering actuator (36) have different metering behaviours (56) for the medical gas (4).

12. Device (2) according to any of claims 9 to 11 above, wherein the metering actuator (36) comprises a throttle orifice (70) connected in series with the switching means (62, 66).

13. Computer program including program code means for performing all steps of a method of controlling a metering actuator (36) in an apparatus (2) according to any of the preceeding claims when the computer program is executed on a computer or one of the specified apparatuses, comprising the steps:
- Detecting a profile (40) of a characteristic (30) of the respiratory gas flow (10, 18) in the respiratory gas channel (12, 20),
- Determining a fill level of a patient's lung based on the acquired profile (40),
- Generating the control signal (34) as a function of the determined filling level.

14. Control device (2) arranged to perform a method for controlling a metering actuator (36) in a device (2) according to one of claims 1 to 12, comprising the steps of:
- Detecting a profile (40) of a characteristic variable (30) of the respiratory gas flow (10, 18) in the respiratory gas channel (12, 20),
- Determining a fill level of a patient's lung based on the acquired profile (40),
- Generating the control signal (34) as a function of the determined filling level.

15. Computer program product containing a program code which is stored on a computer-readable data carrier and which, when executed on a data processing device, performs a method of controlling a metering actuator (36) in a device (2) in accordance with one of claims 1 to 12, comprising the steps:
- Detecting a profile (40) of a characteristic quantity (30) of the respiratory gas flow (10, 18) in the respiratory gas channel (12, 20),
- Determining a fill level of a patient's lung based on the acquired profile (40),
- Generating the control signal (34) as a function of the determined filling level.

## Revendications

1. Dispositif (2) permettant d'appliquer un gaz médical (4) à un patient (6), comprenant :
- un canal de gaz respiratoire (12, 20) adapté pour conduire un flux de gaz respiratoire (10, 18) vers le patient (6),
- un canal d'application (22) conduisant le gaz médical (4) d'une source de gaz dans le flux de gaz respiratoire (10, 18),
- un actionneur de dosage (36) dosant le gaz médical (4) dans le canal d'application (22) en fonction d'au moins un signal de commande (34), et
- un dispositif de détection (28) qui est agencé pour détecter un profil (40) d'une quantité caractéristique (30) du flux de gaz respiratoire (10, 18) dans le canal de gaz respiratoire (12, 20),
**caractérisé par**
- un dispositif de commande (32) agencé pour déterminer un degré de remplissage du poumon d'un patient sur la base du profil détecté (40) et pour générer le signal de commande (34) en fonction du degré de remplissage.

2. Dispositif (2) selon la revendication 1, dans lequel le dispositif de détection (28) comprend un capteur de débit pour détecter un débit du flux de gaz respiratoire (10, 20) comme quantité caractéristique.

3. Dispositif (2) selon la revendication 2, dans lequel le dispositif de commande (32) est agencé pour émettre le signal de commande () basé sur une fusion du profil détecté (40) avec au moins un autre élément d'information décrivant une inspiration du patient (6).

4. Dispositif (2) selon l'une des revendications précédentes, dans lequel le dispositif de commande (32) est agencé pour déterminer une condition (47) pour la quantité caractéristique (30) du flux de gaz respiratoire (10, 18) sur la base du profil (40) et pour générer le signal de commande (34) en fonction d'une comparaison de la condition déterminée (47) et de la quantité caractéristique (30) du flux de gaz respiratoire (10, 18).

5. Dispositif (2) selon la revendication 4, dans lequel le dispositif de commande (32) est agencé pour déterminer la condition (47) en relation avec la respiration du patient (6) dans une période (42) du flux de gaz respiratoire (10, 18) et pour utiliser la condition déterminée (47) dans une période ultérieure (42) du flux de gaz respiratoire (10, 18) pour générer le signal de commande (34).

6. Dispositif (2) selon l'une des revendications précédentes, comprenant :
- un canal d'expiration (20) disposé de manière à éloigner le flux de gaz respiratoire (10, 18) du patient, et
- un autre dispositif de détection (72) qui est agencé pour détecter un contenu (74) de gaz médical (4) ou un produit de réaction du gaz médical (4) dans le flux de gaz respiratoire (18) par le canal d'expiration (20),
- dans lequel le moyen de commande (32) est agencé pour générer le signal de commande (34) en fonction de la teneur détectée (74) du gaz médical (4) dans le flux de gaz respiratoire (18) par le canal d'expiration (20).

7. Dispositif (2) selon la revendication 6, dans lequel le dispositif de commande (32) est agencé pour générer le signal de commande (34) de telle manière que la quantité de gaz médical (4) dans le flux de gaz respiratoire (18) à travers le canal d'expiration (20) est ajustée à une valeur souhaitée prédéterminée.

8. Dispositif (2) selon la revendication 7, dans lequel le canal de gaz respiratoire (12, 20) est un canal d'inspiration (12) conduisant le flux de gaz respiratoire (10, 18) vers le patient (6) et le dispositif de commande (32) est agencé pour ajuster la valeur souhaitée en fonction d'une comparaison de la teneur (74) du gaz médical (4) dans le flux de gaz respiratoire (18) à travers le canal d'expiration (20) et d'une quantité du gaz médical (4) dans le flux de gaz respiratoire (10) à travers le canal d'inspiration (12).

9. Dispositif (2) selon l'une des revendications précédentes, dans lequel le dispositif de commande (32) est agencé pour délivrer le signal de commande (34) sous forme d'une impulsion (48) et l'actionneur de dosage (36) comprend au moins un moyen de commutation (62, 66) agencé pour ouvrir le canal d'application (22) sur la base de l'impulsion (48) pendant une période de commutation prédéterminée (58).

10. Dispositif (2) selon la revendication 9, dans lequel l'actionneur de dosage (36) comprend une batterie de moyens de commutation (62, 66), et le dispositif de commande (32) est agencé pour délivrer le signal de commande (34) de manière multidimensionnelle pour l'activation individuelle des moyens de commutation individuels (62, 66) de la batterie.

11. Dispositif (2) selon la revendication 10, dans lequel les moyens de commutation individuels (62, 66) de l'actionneur de dosage (36) ont des comportements de dosage (56) différents pour le gaz médical (4).

12. Dispositif (2) selon l'une quelconque des revendications 9 à 11 précédentes, dans lequel l'actionneur de dosage (36) comprend un orifice d'étranglement (70) connecté en série avec les moyens de commutation (62, 66).

13. Programme informatique comprenant des moyens de code de programme pour exécuter toutes les étapes d'un procédé de commande d'un actionneur de mesure (36) dans un dispositif (2) conformément à l'une quelconque des revendications précédentes lorsque le programme informatique est exécuté sur un ordinateur ou l'un des appareils spécifiés, comprenant les étapes :
- Détecter d'un profil (40) d'une caractéristique (30) du flux de gaz respiratoire (10, 18) dans le canal de gaz respiratoire (12, 20),
- Déterminer un niveau de remplissage du poumon d'un patient sur la base du profil acquis (40),
- Générer du signal de commande (34) en fonction du niveau de remplissage déterminé.

14. Dispositif de commande (2) agencé pour exécuter un procédé pour commander un actionneur de dosage (36) dans un dispositif (2) selon l'une des revendications 1 à 12, comprenant les étapes suivantes :
- Détecter d'un profil (40) d'une variable caractéristique (30) du débit de gaz respiratoire (10, 18) dans le canal de gaz respiratoire (12, 20),
- Déterminer un niveau de remplissage du poumon d'un patient sur la base du profil acquis (40),
- Générer du signal de commande (34) en fonction du niveau de remplissage déterminé.

15. Produit de programme informatique contenant un code de programme qui est stocké sur un support de données lisible par ordinateur et qui, lorsqu'il est exécuté sur un dispositif de traitement de données, exécute un procédé de commande d'un actionneur de dosage (36) dans un dispositif (2) conformément à l'une des revendications 1 à 12, comprenant les étapes
- Détecter d'un profil (40) d'une quantité caractéristique (30) du flux de gaz respiratoire (10, 18) dans le canal de gaz respiratoire (12, 20),
- Déterminer un niveau de remplissage du poumon d'un patient sur la base du profil acquis (40),
- Générer du signal de commande (34) en fonction du niveau de remplissage déterminé.
